# EUROPEAN PATENT SPECIFICATION

(11) **EP 3 886 730 B1**
(45) Date of publication and mention of the grant of the patent: **15.11.2023**
(21) Application number: 20818023.2
(22) Date of filing: 04.06.2020
(51) Int. Cl.: A61B 17/12, A61B 17/00, A61L 27/50, A61L 31/14, A61M 29/00

(54) **SYSTEMS AND DEVICES FOR IMPROVED OCCLUSION AND DELIVERY**
SYSTEME UND VORRICHTUNGEN FÜR VERBESSERTE OKKLUSION UND ABGABE
SYSTÈMES ET DISPOSITIFS POUR UNE OCCLUSION ET UNE DÉLIVRANCE AMÉLIORÉES

(30) Priority: 05.06.2019 US 201962857409 P; 16.03.2020 US 202062989977 P
(43) Date of publication of application: 06.10.2021
(73) Proprietor: Endoshape, Inc., Boulder, CO 80301 (US)
(72) Inventor: ASHURST, Daniel, Boulder, CO 80301 (US); BARKENBUS, Charles, Boulder, CO 80301 (US); CHOI, Steven, Boulder, CO 80301 (US); GEIST, Lee, Boulder, CO 80301 (US); GODSOE, Jeremy, Boulder, CO 80301 (US); KERN, Madalyn, Boulder, CO 80301 (US); RICKERT, Ryan, Boulder, CO 80301 (US); WILLENBRINK, David, Boulder, CO 80301 (US); CARPENTER, Dean, Boulder, CO 80301 (US)
(74) Representative: Kazi, Ilya
(86) International application number: PCT/US2020/036213
(87) International publication number: WO 2020/247695

(56) References cited:
- WO-A1-2018/053314
- US-A1- 2007 142 859
- US-A1- 2009 043 330
- US-A1- 2009 306 702
- US-A1- 2015 257 765
- US-A1- 2016 143 646
- US-A1- 2016 278 785

## Description

### TECHNICAL FIELD

The subject matter described herein relates to systems, devices, and methods for occluding vasculature in the human body.

### BACKGROUND

During many clinical procedures, a physician requires the reduction or complete stoppage of blood flow to a target region of the patient's body to achieve therapeutic benefit. Occlusive implants are often used for this purpose. Occlusive implants can be used to inhibit blood flow for a wide variety of applications, including the occlusion of blood vessels and the occlusion of aneurysms.

Physicians may be motivated to use occlusive implants for vessel occlusion in order to treat a number of situations, for example, arteriovenous malformations (AVMs), traumatic fistulae, some aneurysm repair, uterine fibroid and tumor embolization. For these clinical treatments, the blood flow through a target section of a blood vessel must be occluded (i.e., significantly reduced or stopped altogether). The delivered implant induces an initial reduction of blood flow through a simple mechanical blockage, which in turn triggers the body's natural clotting process to form a more complete blockage comprised of the thrombus adhered to the implant.

An aneurysm often takes the form of a relatively localized, blood-filled bulge in a weakened wall of a blood vessel. Aneurysms can occur in any arterial blood vessel, with examples including cerebral aneurysms, aortic aneurysms affecting the thoracic aorta, and abdominal aortic aneurysms. As an aneurysm increases in size, the risk of rupture increases. A ruptured aneurysm can lead to bleeding and subsequent hypovolemic shock, which in turn can lead to death. Physicians may treat aneurysms by implantation of one or more occlusive implants within or over the aneurysm. The occlusive implant would then cause a thrombus to form and remain within the confines of the aneurysm, which in turn can decrease the risk of rupture and promote the healing response. In many cases, aneurysms treated in such a manner are almost entirely healed within a manner of months or weeks.

Occlusive implants are typically delivered to the vessel or aneurysm with a sterile catheter percutaneously inserted into the body and routed through the subject's vasculature to the target site. The occlusive implant can be pushed out of an open distal end of the catheter, using a slidable pusher within the catheter, into the aneurysm or vessel. Once deployed in the body, the implant can mechanically inhibit blood flow and promote thrombus formation on or around the implant until the vessel or aneurysm is fully occluded.

WO2018/053314 provides a medical system, comprising: an occlusive implant comprising a flexible body with a covering that comprises a plurality of fibres in the form of a braid, wherein at least one fibre of the plurality of fibres extends from the covering in the form of a loop at a proximal end of the occlusive implant; an elongate tubular member having an inner lumen; and an elongate pusher member slidable within the inner lumen and having a distal end region with a recess adapted to releasably hold the at least one fibre in the form of the loop.

It would be desirable, therefore, to provide improved systems, devices and methods of occlusion of the vasculature within the human body.

### SUMMARY

The invention is defined in independent claim 1. Certain optional features of the invention are defined in the dependent claims. Provided herein are exemplary embodiments of systems, devices, and methods related to vascular occlusion. Embodiments may include an occlusive device that includes an elongate polymer body including a fiber formed in a braid or knitting coupled thereto, and a shape memory wire coupled with the polymer body. The shape memory wire can be secured to the elongate polymer body and can have an instilled shape. The shape memory wire can may be formed of a non-straight instilled shape such as a helix, a coil, a zig-zag shape, or any other suitable shape. In another embodiment, the polymer body may be coupled to a shape memory polymer, with a fiber coupled thereto. The braid may include a first and second fiber, and the shape memory wire can be disposed between the first and second fiber. The shape memory wire can be interlaced or woven into the braid. The elongate polymer body, braid, and shape memory wire can be secured together with at least one band, clamp, or tubular element.

The shape memory wire may be in contact with, and alongside, the elongate polymer body. The braid may wrap around the shape memory wire and the elongate polymer body and may cover at least a portion of the length of the elongate polymer body. The braid may cover at least a partial length, a majority of the length, or the entire length of the elongate polymer body.

The shape memory wire may extend at least a partial length, a majority of the length, one or more segments along the length, or the entire length of the elongate polymer body. The shape memory wire may be formed of a nickel -titanium alloy, shape memory alloy, or nitinol wire. The shape memory wire may be formed of a first portion and a second portion, with each portion pre-shaped to have a first and a second shape, respectively.

In some embodiments, the first and second shapes may be different shapes than one another. In other embodiments, the first and second shapes may be identical shapes. The shape memory metal alloy may include a first portion having a first cross-section and a second portion having a second cross-section. The first and second cross-sections may be of the same or different sizes.

The occlusive device may include an engagement structure at a proximal end of the occlusive device. The engagement structure may include a tube. The tube may be coupled to the braid with a hitch knot or any other suitable knot. Thus, the tube may be coupled to an implant via fiber cover to create a flexible junction. This may decrease the likelihood of detachment, and allows for increased ease of delivery. The tube may formed of polymer, which is then coupled to the braid with a girth hitch knot. The engagement structure may be coupled to the braid with a fiber of the braid.

The polymer body may be made from a single polymer strand or a plurality of polymer strands. The polymer body can be radiopaque. The braid can include one or more fibers along a length of the braid, where the one or more fibers are configured to induce thrombosis.

Also disclosed herein is a delivery system that includes: an occlusive device; an elongate tubular member having a lumen configured to receive the occlusive device; and an elongate pusher member. The elongate pusher member may include a shape memory metal alloy wire. The occlusive device can include: an elongate polymer body having braid coupled thereto; and a shape memory wire coupled with the polymer body.

Also disclosed herein is a method of delivering an occlusive implant. The method includes positioning an open distal end of an elongate tubular member in proximity to a target site; and deploying the occlusive implant from the elongate tubular member to the target site. The occlusive implant can include an elongate polymer body, a braid, and a shape memory wire.

Also disclosed herein is a second method of delivering an occlusive implant. The method includes: positioning an open distal end of an elongate tubular member in proximity to a target site; deploying the first occlusive implant from the elongate tubular member to the target site; and deploying one or more second occlusive implants to the target site using the elongate tubular member. The first occlusive implant includes a first elongate polymer body, a first braid, and a shape memory wire. The one or more second occlusive implants include a second elongate polymer body and a second braid with no shape memory wire.

Also disclosed herein is a method to approximate positioning of the occlusive implant within an elongate tubular member. The pusher member of the delivery system may include markings along the length to create visual and/or tactile indicators. The positioning of these indicators relative to the operator-facing end of the elongate tubular member allows the operator to approximate the position of the occlusive implant within the tubular member. This method of approximating the positioning of the occlusive implant allows shorter procedure times and reduced exposure to radiation from fluoroscopic imaging systems for both the operator and the patient.

The features and advantages described in the specification are not all inclusive and, in particular, many additional features and advantages will be apparent to one of ordinary skill in the art in view of the drawings, specification, and claims. Moreover, it should be noted that the language used in the specification has been principally selected for readability and instructional purposes and may not have been selected to delineate or circumscribe the disclosed subject matter.

### BRIEF DESCRIPTION OF THE DRAWINGS

The foregoing summary, as well as the following detailed description, is better understood when read in conjunction with the accompanying drawings. The accompanying drawings, which are incorporated herein and form part of the specification, illustrate a plurality of embodiments and, together with the description, further serve to explain the principles involved and to enable a person skilled in the relevant art(s) to make and use the disclosed technologies.
Figure 1 is a perspective view depicting an example delivery system in accordance with some embodiments of the present disclosure.
FIGS. 2A-2C and 2G are perspective views depicting examples of occlusive implants in accordance with some embodiments of the present disclosure.
FIGS. 2D-2F are cross-sectional views of occlusive implants in accordance with some embodiments of the present disclosure.
FIG. 3A is a process flow chart for delivering occlusive implants in accordance with some embodiments of the present disclosure.
FIGS. 3B-3C are side views illustrating occlusive implants being delivered via a catheter in accordance with some embodiments of the present disclosure.
FIG. 4 is a perspective view of a pusher with radial slots in accordance with some embodiments of the present disclosure.
FIG. 5 is a side view of an example pusher member in accordance with some embodiments of the present disclosure.
FIG. 6 is a close-up perspective view of a proximal portion of an occlusive implant in accordance with some embodiments of the present disclosure.
FIGS. 7A-B are perspective and side views, respectively, of an engagement structure of an occlusive implant in accordance with some embodiments of the present disclosure.
FIGS. 8A-B are side views illustrating occlusive implants being captured by a pusher member in accordance with some embodiments of the present disclosure.
FIG. 8C is a close-up view of an occlusive implant being captured by a pusher member within a catheter in accordance with some embodiments of the present disclosure.
FIGS. 9A is a side view of a transfer/removal tool in accordance with some embodiments of the present disclosure.
FIG. 9B is an exploded side view illustrating the transfer/removal tool of FIG. 9A.
FIG. 9C is a side view illustrating a spring-loaded transfer/removal tool coupled with a catheter hub in accordance with some embodiments of the present disclosure.
FIGS. 10A-10C illustrate an exemplary polymer body with different shapes along the length, and a cross sectional view of a vessel with an implant packed tightly.

The figures and the following description describe certain embodiments by way of illustration only. One skilled in the art will readily recognize from the following description that alternative embodiments of the structures and methods illustrated herein may be employed without departing from the principles described herein. Reference will now be made in detail to several embodiments, examples of which are illustrated in the accompanying figures. It is noted that wherever practicable similar or like reference numbers may be used in the figures to indicate similar or like functionality.

### DETAILED DESCRIPTION

The present subject matter is described in the context of the use of an occlusive implant that can take the form of a coil, which is implanted within the vasculature (e.g., a blood vessel or aneurysm) to obstruct blood flow within or to that vasculature. The present subject matter is not limited only to implants that can take the form of a coil, as the subject matter is similarly applicable to implants that have either multiple coils or structures or forms other than those of a coil. Likewise, the present subject matter is not limited only to the occlusion of the vasculature (e.g., peripheral vessel occlusion) as the subject matter is applicable to the treatment of many types of disease where passive or active release of an implant is desirable, including but not limited to the treatment of septal defects in the heart, the treatment of left atrial appendages, and the like.

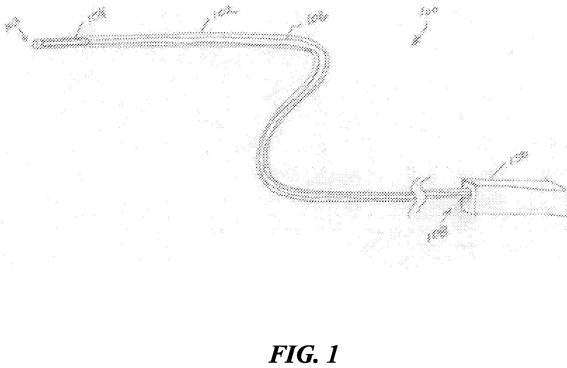

FIG. 1 is a perspective view of an example embodiment of implant delivery system 100, which can be used with all embodiments described herein. System 100 includes an elongate tubular member, which can be configured as a catheter, a micro-catheter, or a sheath. Tubular member 102, which for ease of discussion will be referred to as catheter 102, can be percutaneously introduced into a patient's vasculature and then advanced to a target treatment site, either directly or with the aid of a guidewire. In some embodiments, tubular member 102 can slide within a larger tubular member, such as a guide catheter (not shown).

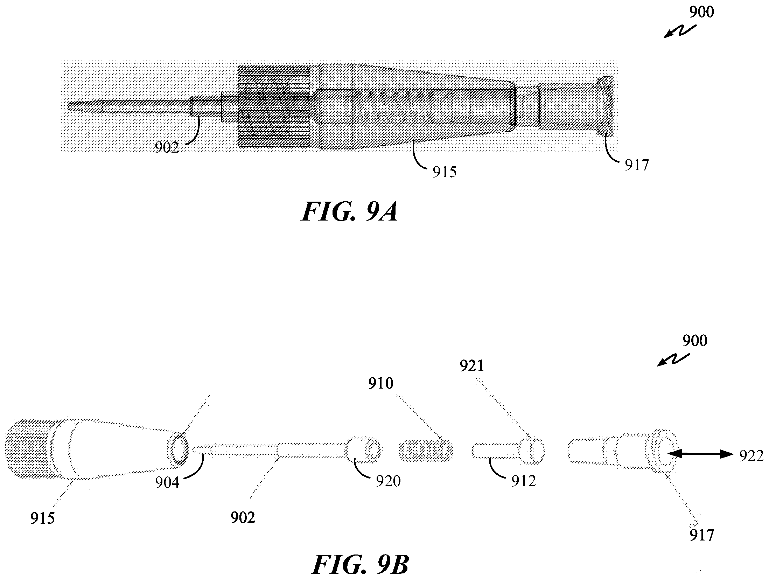

Catheter 102 includes an open distal end 103 from which an occlusive implant 104 can be delivered. Occlusive implant 104 is slidable within catheter 102 and can be advanced with an elongate pusher member (referred to herein for brevity as a pusher) 106 that is also slidable within catheter 102. A proximal end 108 of catheter 102 can be coupled with a proximal device 150 that resides outside of the patient and can include one or more interfaces (not shown) for use by the medical professional to accomplish or control the delivery (implantation) procedure. Proximal control device 150 can also have one or more ports for introducing components of system 100 and flushing. Proximal control device 150 be configured as (or include) the embodiments of transfer tools described with respect to FIGs. 9A-10B herein.

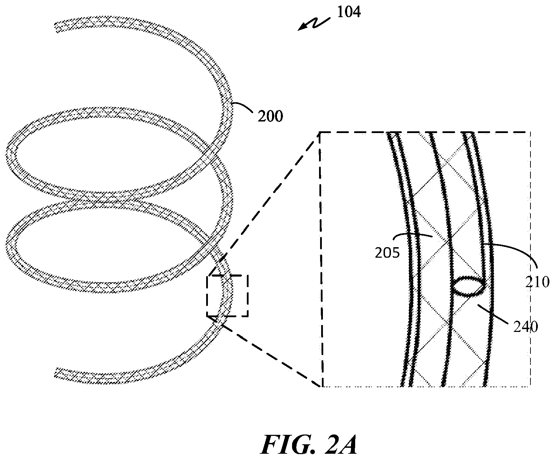

FIG. 2A illustrates implant 104 in accordance with some embodiments of the present disclosure. Implant 104 can include an elongate polymer body 205 with a covering 200, and an elongate wire 210. Covering 200 may be formed of a fiber structure that is braided, or knitted.

Both knit or braided coverings may each provide a tubular fiber outer structure for covering polymer body 205. Both knit or braid structures can be comprised of multiple filaments. Multiple filaments provide the ability to select multiple materials to tune the properties of the implant, for example low friction materials, shape memory or high thrombogenicity.

In some embodiments, the braided cover may constrain the polymer body 205. This provides for tuning the implant stiffness, which is advantageous for forming longer implant lengths. The braid may be formed of multiple fiber strands. The multiple fiber strands may be a mix of different distinct fiber materials. The mix of fiber materials provides the ability to produce reduced surface friction and higher thrombogenicity properties. The braid structure also allows the production for a seamless proximal loop. Thus, for example, fibers within the braid structure may be utilized to create a loop at a proximal end of the braid, using one of the braid lines. The loop may be seamlessly integrated into the braid structure to provide a small feature with high tensile strength.

In some embodiments, the knit structure utilizes an interconnected loop structure that can be either locked in place or not, which provides the ability to control the radial and axial expansion. This structure can allow for high implant conformance, while also reducing the restriction of the implant. Such conformance is particularly advantageous for certain applications, such as the occlusion of smaller vessels or aneurysms, such as, for example, less than 3 millimeters in diameter, or any other suitable diameter. Reduced restriction on the implant may allow the implant to have greater shape recovery. The knit may be formed of a single fiber strand. In other embodiments, the knit may be formed of multiple fiber strands.

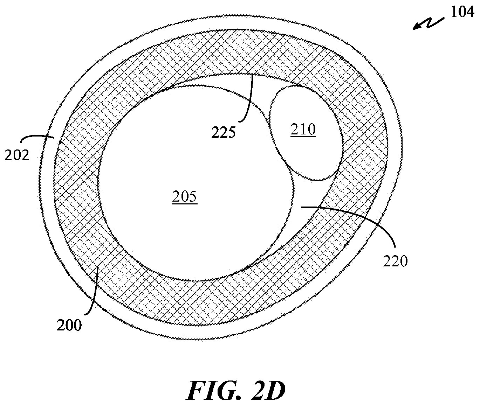

Body 205 and wire 210 are obscured by cover 200, but can be seen in the closeup where cover 200 is made partially transparent. The various locations of polymer body 205 and elongate wire 210 with respect to each other and to cover 200 will be discussed below with respect to FIGS. 2D-2F. Polymer body 205 can be only a single strand of polymer or can be multiple strands of polymer, e.g., arranged side-by-side as a bundle. Polymer body 205 can include one or more medical grade polymers or copolymers. While not required, polymer body 205 is preferably a radiopaque polymer. Examples of suitable radiopaque polymers are described in US Publ. No. US 2013/0225778, US Publ. No. 2015/0374884, and US Publ. No. 2016/0024239.

Covering 200 may be formed by crossing multiple fibers over polymer body 205 resulting in a braided structure cover 200 can be braided in many desired formats, such as a 1×1, 2×1, 2x2, and the like. A braid may be comprised of three or more lines, and the braid angle may be varied with the PPI (pics per inch) thereby changing the constraint of the fibers on the polymer body 205. The use of cover 200 can be advantageous in that it reduces surface friction between body 205 and an interior surface of catheter 102 if a low friction fiber material is used. The tight constraints of the anatomy impose even tighter constraints on the diameter of the inner lumen of catheter 102. For example, a typical 3 French catheter 102 may have an inner diameter of 0.027 inches, (0.69mm) while a 4-5 French catheter may have an inner diameter of 0.038 inches (0.96mm). These small diameters, coupled with the significant length of implant 104, may result in large surface area of implant 104 that contacts the inner surface of catheter 102. This can result in a high surface friction between implant 104 and catheter 102. This can be especially true if catheter 102 and the polymer body 205 are both polymeric surfaces. The ability for the implant 104 to slide within the catheter 102 can be substantially hindered when the catheter is negotiating tortuous bends that are often encountered in the anatomy. A reduced surface friction will more easily allow the medical professional to advance the implant from within catheter 102, and thus increased the length of implant that can be used. Braid can also affect flexibility and other performance characteristics.

The use of cover 200 may lower this surface friction considerably. It has been found that polymeric materials having sufficient flexibility such as ultra-high-molecular-weight polyethylene (UHMWPE), polytetrafluoroethylene (PTFE), fluorinated ethylene propylene (FEP), or other polypropylenes, when used for cover 200 can significantly reduce the surface friction on the implant 104, when moving through catheters formed from polyether block amide (PEBAX), nylons, polyurethanes, or thermoplastic elastomers, with or without fluorinated polymer internal linings. The presence of cover 200 also increases the tensile strength of the overall implant itself, making the implant less subject to stretching. UHMWPE, for example, can also significantly increase the tensile strength of the implant and reduce the coefficient of friction.

Elongate wire 210 may be secured to polymer body 205 at one or more locations along the length of polymer body 205. Elongate wire 210 can be loosely or tightly secured to polymer body 205. In some embodiments, elongate wire 210 can be secured to polymer body 205 for the entire length of polymer body 205 or elongate wire 210, whichever is shorter in length. Elongate wire 210 can be secured to polymer body 205 using a band, a clamp, a tubular element (e.g., rubber band tube), glue, or a combination thereof. Elongate wire 210 can be also secured to polymer body 205 using a plurality of bands, clamps, tubular elements, or a combination thereof (e.g., a plurality of bands and clamps).

Cover 200, polymer body 205, and elongate wire 210 can also be secured together using a one or more bands, clamps, tubular elements, glue, caps, or a combination thereof. Elongate wire 210 can also be interlaced between interstitial openings of cover 200. In some embodiments, cover 200 be wrapped around polymer body 205 and elongate wire 210. Cover 200 can be wrapped around polymer body 205 and elongate wire 210 at one or more portions of polymer body 205 and elongate wire 210.

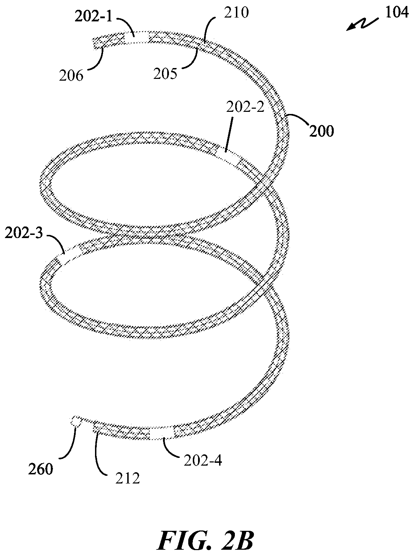

Implant 104 can include any number of one or more radiopaque bands. FIG. 2B illustrates an example embodiment of implant 104 with four radiopaque bands 202-1 through 202-4. In some embodiments, polymer body 205 and elongate wire 210 can be positioned next to each other in contact within cover 200. FIG. 2D illustrates a cross-sectional view of the embodiment of FIG. 2B taken across implant 104 at the position of a radiopaque band 202, wherein both polymer body 205 and wire 210 are within cover 200. A variable amount of space 220 may be present between cover 200 and body 205 and wire 210. Cover 200 can, in some embodiments, closely conform to the surface of body 205 and wire 210 such that the space 220 is minimized, or there can be additional space 220 as depicted in FIG. 2D. In this embodiment, both polymer body 205 and elongate wire 210 are completely encompassed by cover 200. Radiopaque band 202 can be fastened (e.g., crimped, adhered, and the like) to securely hold cover 200, polymer body 205 and elongate wire 210 together. In some embodiment, bands 202 can be made from a non-radiopaque material, and any radiopacity of polymer body 205 can be used for visualization.

These radiopaque bands 202 may be positioned at equidistant intervals along the length of implant 104, variable different intervals, or any combination of the two. For example, bands 202-1 and 202-2 can be disposed near each other and near distal end 206 of implant 104. Similarly, bands 202-3 and 202-4 can be disposed near each other and close to proximal end 212 of implant 104. In some embodiments, at least one band is at or near distal end 206 and at least another band 202 is at or near proximal end 212. Here, band 202-1 is in proximity with the distal terminus of implant 104, while band 202-4 is in proximity with the proximal terminus of implant 104, and bands 202-2 and 202-3 are positioned in intermediate locations.

In some embodiments, radiopaque (RO) bands 202 may be located at the most distal and most proximal locations, and are also used to clearly indicate the ends of the implant during visualization using fluoroscopy. This is shown for example as 202-1 and 202-4, respectively, in FIG. 2B. Catheter 102may include a radiopaque marker near the distal opening to provide visual indication under fluoroscopy. The pusher 106 may include a RO marker to provide visual indication under fluoroscopy.

In some embodiments, the RO markers may be used to determine the positions of the catheter 102, implant 104, and pusher 106 to complete the process of delivering, deploying, and detaching the implant 104. An exemplary process may include the following steps. At a first step, the catheter 102 is positioned, using the marker band on catheter 102, at a desired location. The implant 104 will then be delivered, defined as the action of advancing the implant 104 into the catheter 102 until the distal end of the implant reaches the distal end of the catheter 102. Implant 104 is then advanced into the catheter 102 without the use of fluoroscopy until at least the entire implant length is fully inside the catheter 102. Fluoroscopy may then be used to visualize the implant marker(s) to locate the implant position and continue to advance the implant until the distal marker of the implant 104 reaches the location of the catheter 102 RO marker. Implant 104 may then be deployed into the desired location by advancing the implant outside the catheter 102 distal tip until the implant 104 proximal marker nears the catheter marker. Detachment of the implant 104 may then occur when the pusher marker is advanced beyond or distal of the catheter 102 marker. At this point, the proximal engagement mechanism on the implant will release from the pusher, which will complete the detachment of the implant.

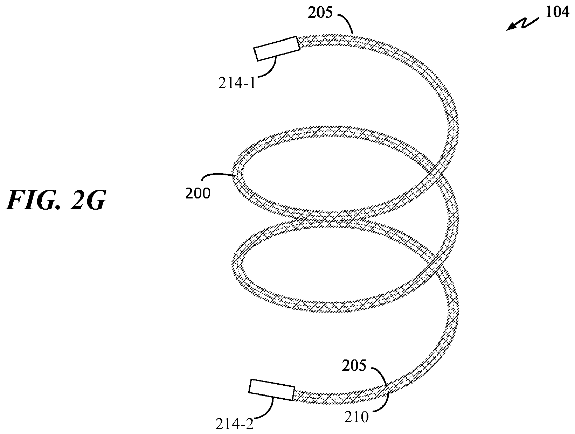

In some embodiments, caps 214-1 and 214-2 may be placed over the distal and proximal ends of implant 104, respectively, instead of a band 202, as depicted in FIG. 2G. Alternatively, only one end of implant 104 can be capped (either proximal or distal end). One or more caps 214 can be used in combination with one or more bands 202. Distal end 206 can be capped and proximal end 212 can be configured with release structure 260. A cap 214 can have a closed end and an open end for capturing wire 210, body 205, and/or cover 200. A cap 214 can also serve as a radiopaque marker.

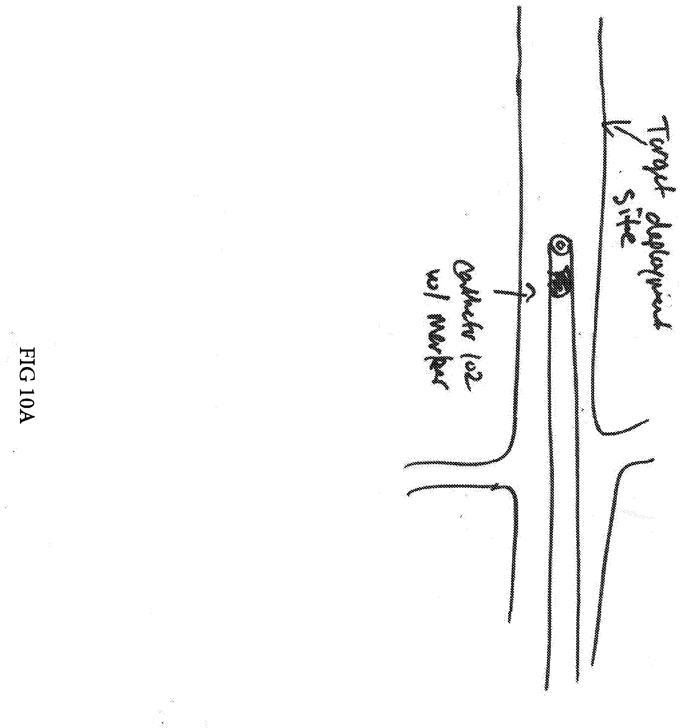

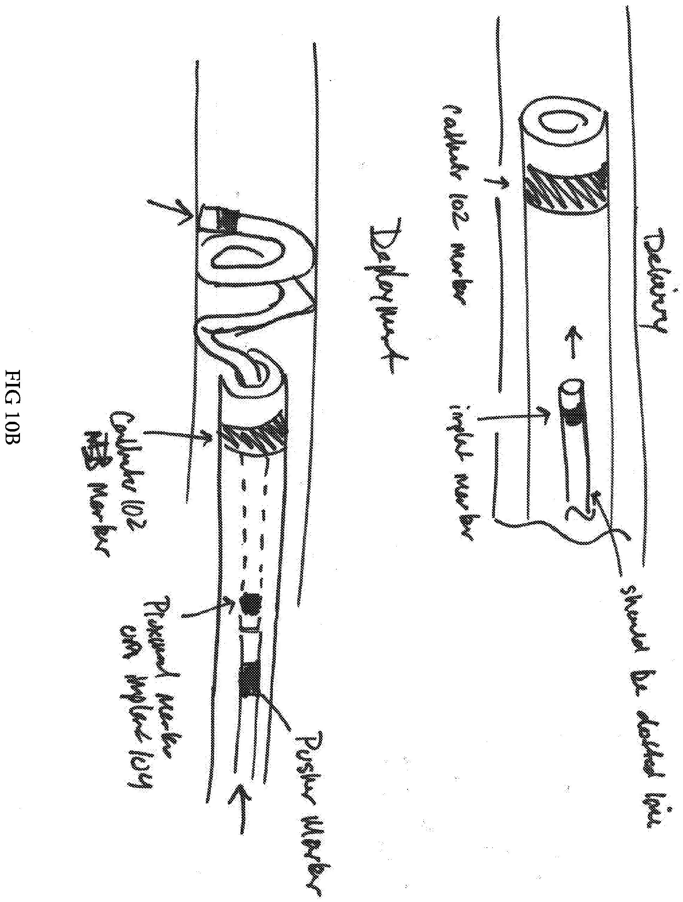

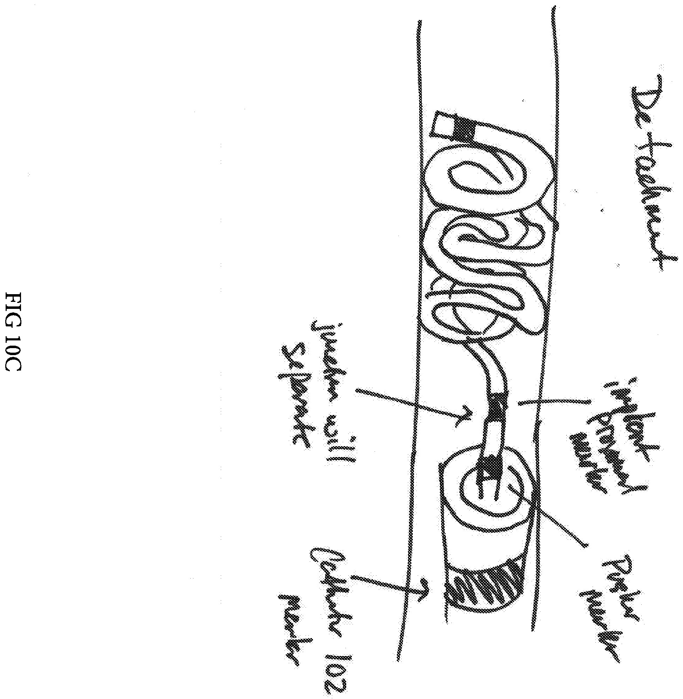

Referring again to FIG. 2D, although polymer body 205 is shown to have a circular cross-section, polymer body 205 can have other cross-sectional shape such as a circle, an oval, or a polygon, as also illustrated in FIGS. 10A-10C. Similarly, elongate wire 210 can have a cross-sectional shape of an oval, a circle, or a polygon. The diameter of polymer body 205 can be bigger than the diameter of elongate wire 210. In some embodiments, the diameter of polymer body 205 can be the same or smaller than the diameter of elongate wire 210.

In some embodiments, implant 104 does not have band 202. Instead, polymer body 205 and elongate wire 210 can be held together by cover 200 or by other means such as, but not limited to, glue, heat shrinkable materials or clamps. For example, polymer body 205 and elongate wire 210 can be adhesively attached to each other, both of which can be disposed inside of void 202 and held together by cover 200. In some embodiments, polymer body 205 and elongate wire 210 can be adhesively attached to inner wall 225 of cover 200.

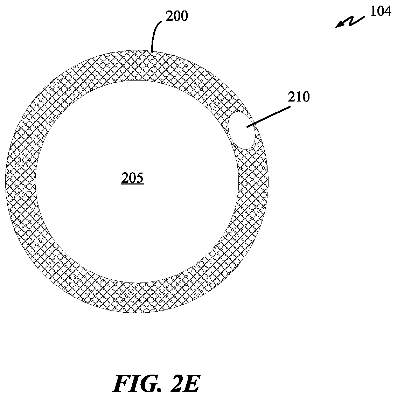

FIG. 2E is a cross-sectional view depicting an example embodiment of implant 104 where elongate wire 210 is woven within cover 200 (a band 202 can be included but is not shown). In this embodiment, elongate wire 210 can be secured to cover 200 by weaving or interlacing elongate wire 210 through cover 200. For example, two or more strands of fiber can wrap around elongate wire 210 in a crossing pattern to form a braid that captures elongate wire 210. For instance, elongate wire 210 can be woven between one or more fibers of braid materials such that the entirety of elongate wire 210 is encompassed by cover 200, or such that portions of wire 210 are exposed to the exterior. Elongate wire 210 can be interlaced through the interstitial openings of cover 200 at various locations along the length of cover 200. For example, elongate wire 210 can be woven within cover 200 at one or more portions of cover 200 and inside the lumen of cover 200 (FIG. 2D) at other portions.

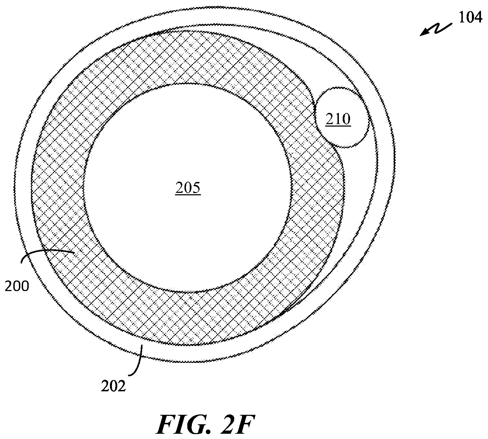

FIG. 2F is a cross-sectional view of implant 104 having elongate wire 210 being coupled to cover 200 by band 202 in accordance with some embodiments. As shown, elongate wire 210 is disposed outside of cover 200 and is held in place by the compressive force of band 202. The approaches of two or more of the embodiments of FIGS. 2D-2F can be combined as well in one embodiment.

Polymer body 205 may be formed of a variable diameter along the length or at different regions along the length. The polymer body may also have distinct sections along the length, formed of differing cross-sectional shapes with different relative orientations. For example, the polymer body may be formed to create a mixture of circular and oval cross-sections such that the oval sections have different planer angles. These sections may be equally repeated along the length or created with random segment lengths. These features can be advantageous for increasing implant conformability by creating varying implant stiffness and creating points of inflection for the implant, to variably pack into a tight conformed space. This tight packing may therefore provide an occlusive mass within a target space with high volumetric density and well distributed cross-sectional coverage across the target space, which may lead to fast acute occlusion in addition to long lasting and durable occlusions.

Implant 104 may or may not utilize the elongate wire 210. The implant 104 can use the elongate wire 210 to aid in shape and radial force extorted by the implant. This can be advantageous in building an occlusive mass that stable inside the target occlusion site. An implant 104 without the elongate wire 210 can be advantageous to increase the conformability of the implant.

Elongate wire 210 can run the entire length polymer body 205. For example, elongate wire 210 can run from proximal end 212 to distal end 206 of implant 104. In some embodiments, elongate wire 210 can extend a partial length of polymer body 205.

Elongate wire 210 can be made of a shape retensive metal, alloy, such as, but not limited to, a nickel-titanium alloy or shape memory alloy. In some embodiments, elongate wire 210 can be a formed from a nickel-titanium alloy such as super elastic Nitinol or shape retensive Nitinol. In addition, polymer body 205 can be made with radiopaque polymers or copolymers, which can further improve the radiopaque property of implant 104.

In some embodiments, the shape retensive characteristic may be activated by body temperature, while in others the implant can return to its shape upon exiting the delivery device. The use of a shape retensive metal enables elongate wire 210 to better retain its shape while also improving radial force when deployed in the vasculature, which enables more predictable anchoring and pack formation. Elongate wire 210 can be a single-strand of material (e.g., super elastic Nitinol). Elongate wire 210 can also comprise two or more Nitinol wires

In some embodiments, a single or multi-filament Nitinol wire can be used to construct elongate wire 210 that extends the length of the implant, alongside a polymer body 205. The Nitinol wire can run from the distalmost marker band of a braided structure to the proximal most marker band of the braided structure. The Nitinol wire can have an instilled shape of a coil, a helical shape, a multi-coil shape, a ball, a polygon, or other non-straight shapes. An instilled shape can be formed on a wire by wrapping the wire around an object (e.g., a mandrel) and then heat treating the wire to impart the shape onto the wire. The wrapping patterns and the shape of the mandrel can be changed to impart different kind of shapes onto the wire. For example, a hexagonal mandrel can be used, and the wrapping pattern can have a rectangular or circular pattern. The heat setting profile (e.g., temperature, heat exposure time) can vary and depend on the types of metal used.

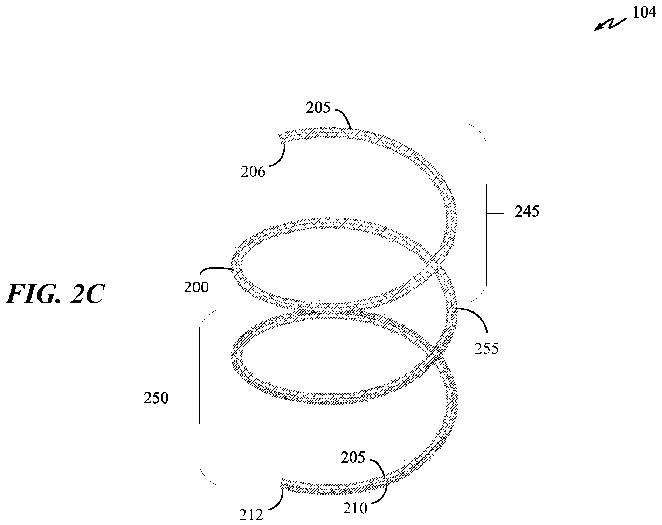

The Nitinol wire can have multiple distinct segments. For example, elongate wire 210 can have two distinct segments 245 and 250 as illustrated in FIG. 2C. The first segment, segment 245 (distal half), of elongate wire 210 can be coupled to polymer body 205 starting midsection 255 of implant 104 and ending at distal end 206 of implant 104. The second segment, segment 250 (proximal half), can coupled to polymer body 205 starting at proximal end 212 to midsection 255 of implant 104. The two separate wire segments can be shape set to a coil or other shapes, and polymer body 205 can have a shape set or can be without a shape set (e.g., can have a low modulus and remains in a free form or floppy state). Segments 245 and 250 can have the same instilled shape or have different instilled shapes. Such a configuration allows the implant to have shape sets in various regions and be devoid of shape sets in other regions. Any number of combinations of wire and polymer body 205 can be implemented with or without this shape set variation (e.g., a wire disposed only in a distal region, only in a middle region, only in a proximal region, any combination thereof, and the like).

The Nitinol wire can also have different diameter regions. For example, segment 245 of elongate wire 210 can have a first diameter, and segment 250 of elongate wire 210 can have a different diameter. For instance, segment 250 can have a larger diameter than the diameter of segment 245. Elongate wire 210 can have a transition area near or at midsection 255 where the diameter transitions from a first to a second diameter. Elongate wire 210 can also have varying dimension at various locations or portions of elongate wire 210.

In some embodiments, the Nitinol wire may also have different cross-sectional size and/or shape at different regions of the wire. Transitions between different cross-sectional sizes and/or shapes can be gradual or tapered instead of immediate or instantaneous transitions. For example, segment 245 can have a circular cross section with a larger diameter than the diameter of the polygonal cross section of segment 250, with a gradual transition between segments 245 and 250. In this way, the flexibility and manipulability of elongate wire 210 can be manipulated by changing its size and shape. Elongate wire 210 can have the same cross section for the entire length of elongate wire 210. In some embodiments, elongate wire 210 can have two or more cross sections. For example, the proximal half (segment 250) of elongate wire 210 near the proximal end can have a first cross-sectional shape and the distal half (segment 245)) of elongate wire 210 can have a second cross-sectional shape. The first and second cross-sectional shapes can be the same or different. For instance, segment 245 can have a square-shaped cross section, and segment 250 can have a polygonal cross section.

In some embodiments, a portion or the entire length of elongate wire 210 can be shaped to retain a certain shape memory. For example, only segment 245 of elongate wire 210 may be pre-shaped to retain a helical shape while segment 250 is not pre-shaped. In another example, segment 245 can have a different cross-sectional shape such as a square, a hexagon, a triangle, etc. In another example, segment 245 can be heat treated to retain a helical shape and segment 250 can be heat treated to retain a spherical shape.

Elongate wire 210 can have three or more different segments each segment can have a different cross-section, shape set, and/or diameter. For example, the first and third segments can have a large diameter and second (middle) section can have a smaller diameter. In another example, the first and third segments can have a first shaped cross section and second (middle) section can have a different cross section. In another example, the first and third sections can have the same shape set and the middle section can have a different shape set. In yet another non-exhaustive example, the first section can have a first diameter and shape set, the second section can have a second diameter and shape set. Lastly, the third section can have a third diameter and shape set. Each of the first, second, and third diameters and shape sets can be the same or different. Additionally, one or more of the first, second, and third diameters and/or shape sets can have the same size and shape.

In some embodiments, one or more of the strands of fiber from cover 200 (or elsewhere) can extend from and beyond proximal end 212 to form engagement structure 260, which can be a knot or ball of fibers. Structure 260 can also be formed by attaching a solid structure to the one or more strands of fiber that extend from proximal end 212. Thus, a tube may be coupled to an implant via fiber cover to create a flexible junction. This may decrease the likelihood of detachment, and allows for increased ease of delivery. The solid structure can be a coil of nickel-titanium (e.g., Nitinol) wire, a ball of fibers, a tube, or any other suitable bio-compatible solids with appropriate physical characteristics and dimensions. Structure 260 is adapted to engage with a corresponding recess at the distal portion of pusher 106. This allows pusher 106 and structure 260 to be releasably attached with each other.

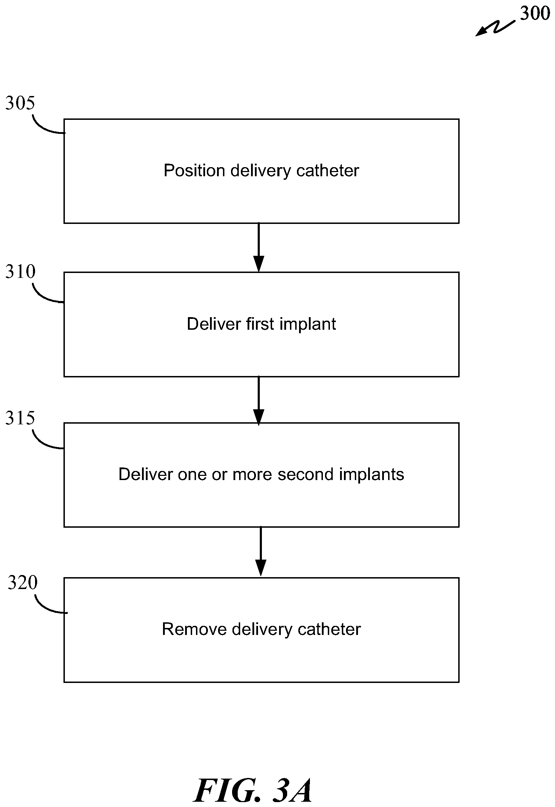

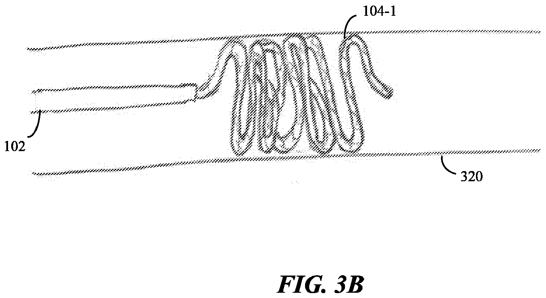

FIGS. 3A-3C will now be discussed concurrently. FIG. 3A is a flow chart illustrating a delivery process 300 of one or more occlusive implants. FIGS. 3B-3D are partial cross-sectional views depicting an example delivery of a primary implant 104-1 followed by secondary implants 104-2 and 104-3 into a blood vessel 320 in accordance with some embodiments of the present disclosure. At 305, catheter 102 is inserted into vessel 320 (see FIG. 3B). Once at the intended delivery site in vessel 320, a pusher is advanced through catheter 102 to advance primary implant 104-1 into vessel 320 (block 310 of FIG. 3A, also FIG. 3B). Implant 104-1 can be configured with a polymer body, braid, and/or wire in accordance with any and all embodiments described herein. Once freed from the constraint of catheter 102, implant 104-1 can take a pre-defined shape within the wall of vessel 320 if configured to do so by wire (not shown), else implant 104-1 can assume a generally random shape. Preferably, implant 104-1 has an instilled shape (e.g., a helical coil or other 2D or 3D shape) and can form a framing structure within vessel 320.

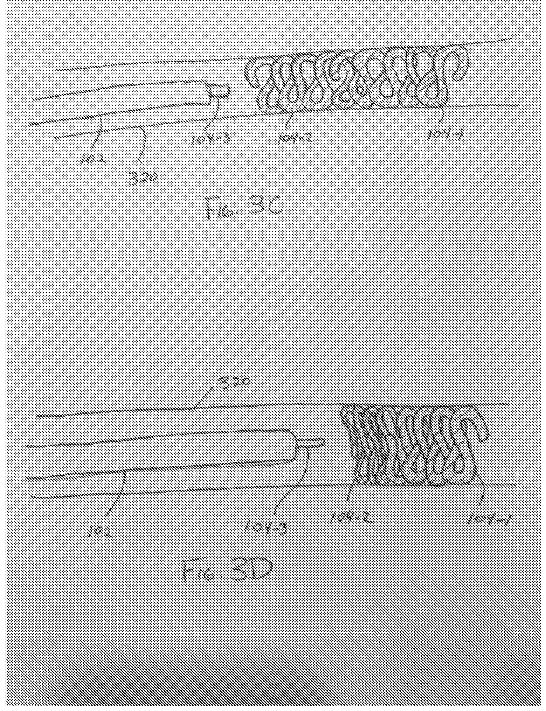

Using the same catheter 102 (or a different catheter), one or more secondary implants 104-2, 104-3 can be loaded and delivered proximally behind implant 104-1 (step 315 of FIG. 3A). FIG. 3C depicts an example where primary implant 104-1 is deployed in vessel 320, followed by secondary implant 104-2. Another secondary implant 104-3 is shown upon initial exposure from catheter 102. In FIG. 3C, all implants 104-1 through 104-3 are of similar size (e.g., diameter and length). In FIG. 3D, primary implant 104-1 has a relatively larger diameter than secondary implants 104-2 and 104-3. In some embodiments, after implant 104 is delivered, the pusher is completely removed ( in order to advance the next implant 104 through and from within the lumen of catheter 102.

One or more secondary implants 104 can be delivered inside or immediately behind the framing structure formed by the primary implant 104. The secondary implants 104 can be configured similar to implant 104 with or without wire 210, allowing the secondary implants 104 to assume a necessary shape that will fill openings within framing implant 104, or that will allow creation of a packed mass of secondary implants 104 behind the primary implant 104. Many secondary implants 104 can be delivered to pack the area within the primary implant 104.

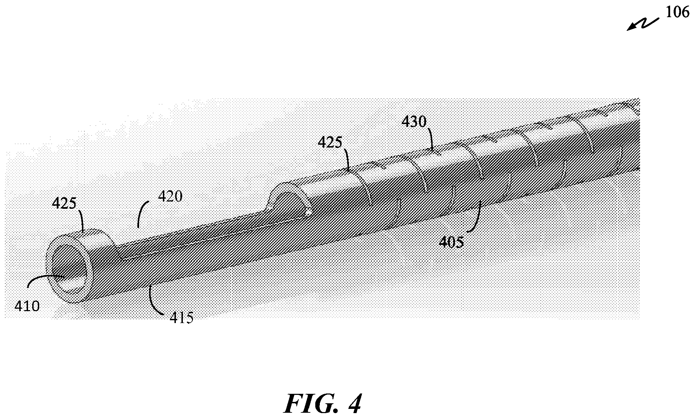

FIG. 4 is a perspective view of pusher 106 in accordance with some embodiments of the present disclosure. Pusher 106 can be made from a hollow or solid wire. In some embodiments, pusher 106 can be made from a tube 405 with a lumen 410. Tube 405 can be made of metal (platinum) or metal alloy such as, but not limited to, stainless steel. Lumen 410 can stretch the entire length of tube 405. Tube 405 includes a distal portion 415 and a proximal portion (not shown), which is near proximal control device 150 (FIG. 1). Distal portion 415 includes a slot 420 adapted to catch or snag structure 250. Slot 420 can have various shapes. For example, from a sideview (lengthwise) perspective of pusher 106, slot 420 can have a rectangular shape. Slot 420 can also have other shapes such as, but not limited to, a square, a circle, or an inverted triangle.

Pusher 106 may also include a plurality of radial slots 425 and 430. Radial slots 425 and 430 are cut into tube 405 and can be distributed along a portion or the entire length of pusher 106 (except where slot 420 is located. Radial slots 425 and 430 can have different sizes, shapes, length, and pitch, which is the angle of the cut. Radial slots 425 and 430 can be distributed according to a pattern such as an alternating pattern or every two or three slot 425 to one slot 430. Each slot can be at straight into the tube at a 0° degree. Each slot can also be cut at an angle such as 15° or 20° degrees. In some embodiments, each radial slot can be cut at an angle of 10 degrees. Each radial slot can also run up to three quarter of tube 405 circumference.

Slot 420 and radial slots 425 and 430 may be cut from a single tube 405 using various cutting methods such as, but not limited to, electrical discharge machine (EDM), grinding, and laser.

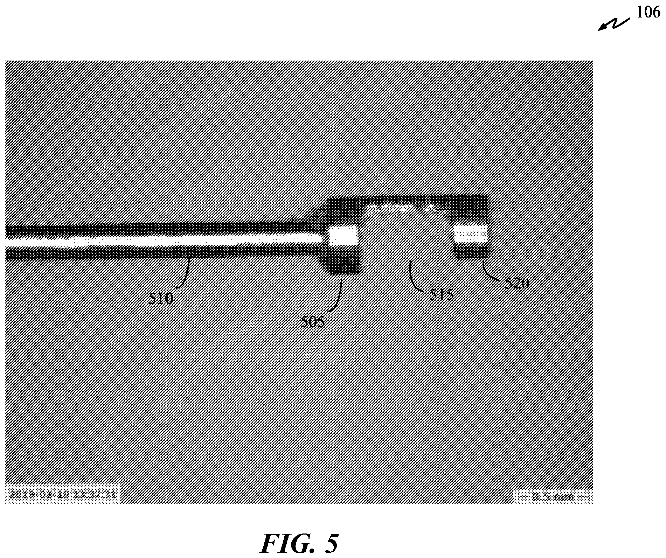

FIG. 5 illustrates a pusher 106 in accordance with some embodiments of the present disclosure. Pusher 106 includes a distal portion 505 with a diameter larger than the diameter of the main wire (e.g., tube) 510. Pusher 106 can also include a distal end portion 520 that has a larger diameter than main wire 510 but smaller than distal portion 505. Distal portion 520 can have the same diameter as distal portion or main wire 510.

Distal portion 505 also includes slot 515, which can have different shapes such as a half-circle, a rectangle, an inverted triangle, etc. Slot 515 is adapted to catch or ensnare engagement structure 260 (e.g., FIG. 2B) at the proximal end of occlusive implant 104. Similar to pusher 106, pusher 106 can be made from a single piece of material or wire. Pusher 106 can also include slots (e.g., grooves) cut into the main body of main wire 510. Different shapes, sizes, and patterns slots can be cut onto main wire 510. In this way, the flexibility and rigidity of main wire 510 can be accurately controlled.

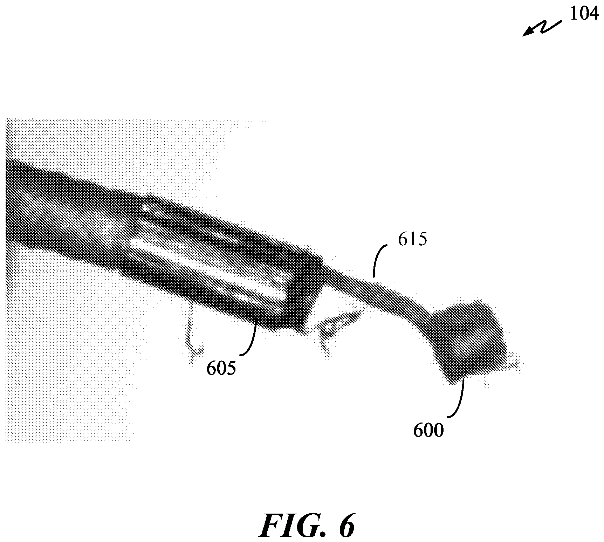

FIG. 6 is a close-up view illustrating engagement structure 260 of occlusive implant 104 in accordance with some embodiments of the present disclosure. As shown, the distal end of occlusive implant 104 includes an engagement structure 260, which can be a ball of fibrous material, a semi-solid, or solid material attached to distal end 605. Engagement structure 260 can be attached to distal end 605 by one or more strands of fiber or wire. In some embodiments, the one or more strands of fiber can be the same fiber that makes up cover 200 that envelops occlusive implant 104. Engagement structure can also be a coil of material instead of a ball.

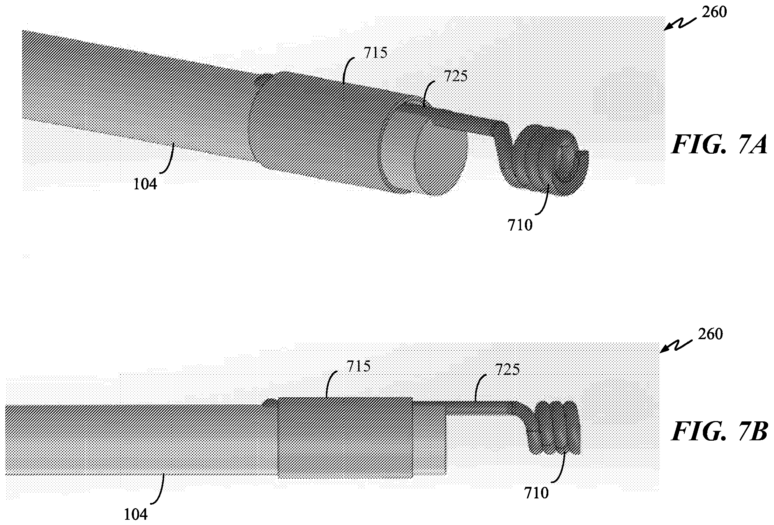

FIG. 7A is a perspective view of the proximal portion of occlusive implant 104 having a wound coil as part of an engagement structure, in accordance with some embodiments of the present disclosure. FIG. 7B is a side view of the proximal portion occlusive implant of FIG. 7A. As shown in FIG. 7A, occlusive implant 104 includes a proximal portion having engagement structure 260. Engagement structure 260 includes a wire coil 710 with an elongate section of the wire 725 that extends distally to the implant body and is secured by band 715. Band 715 a tight wrap of fiber or wire, or an elastic band. Adhesive can be used as an alternative to band 715, or in addition to band 715. In some embodiments, engagement structure 260 can be a coil made of metal (e.g., stainless steel, platinum), metal alloy (e.g., nickel-titanium, stainless steel), or other biocompatible materials. As an alternative to a coil, engagement structure 260 may have other types of structure such as, but not limited to, a spherical, a cylindrical, or a pyramidal structure.

In some embodiments, elongate section 725 may be a portion of cover 200 of occlusive implant 104, formed using one or more fibers from cover 200, which can then be tied or otherwise coupled to coil 710. Elongate section 725 may include a single braid, double braid, a reverse braid, changes in per inch crosses (PIC), or a combination thereof.

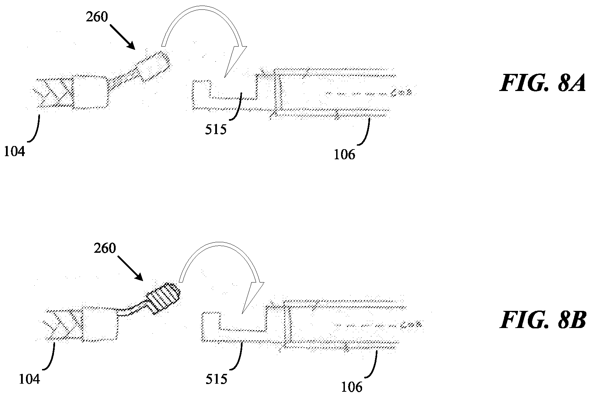

FIGS. 8A and 8B illustrate different versions of an engagement structure (e.g., 260, 600, 700) in the progress of mating with the distal extension (e.g., 415, 505) of a pusher (e.g., 106). For example, FIG. 8A illustrates how engagement structure 260 can fit into slot 515 of pusher 106. Similarly, FIG. 8B illustrates how engagement structure 700, which has a coil design, can also fit into slot 515

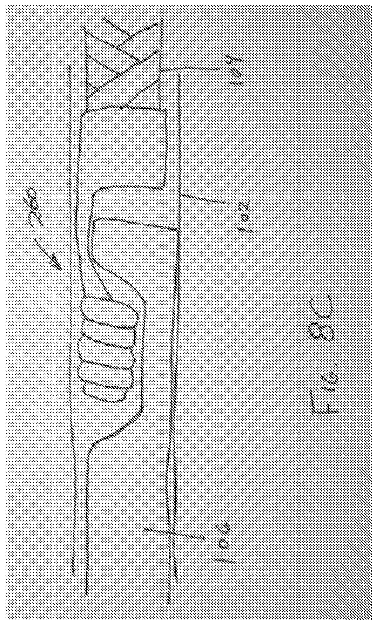

FIG. 8C illustrates occlusive implant 104 and a pusher (e.g., 106, 106) within catheter 102 after occlusive implant 104 is captured by the pusher. Once inside the lumen of catheter 102, occlusive implant 104 is secured by the engagement structure 260 and by the inner wall of catheter 102. During a retrieval process, pusher 106 is pulled toward the proximal end of catheter 102 until occlusive implant 104 is removed. During the implant installation process, pusher 106 is advanced toward the distal end of catheter 102 until both the engagement structure 260 and the distal portion (e.g., 415, 505) of pusher 106 is outside of catheter 102. Once outside the confine of the inner wall of catheter 102, the engagement structure can be released by disengaging the slot of the distal portion. This can be done by rotating pusher back and forth until the engagement structure is released.

As previously mentioned, the slot (e.g., 415, 515) of a pusher 106 can have any shape such as a half circle, an inverted triangle, and a rectangle. Whatever the shape selected for the slot, the engagement structure is selected to have a corresponding male shape that would fit into the slot. For example, if the slot has an inverted triangle shape, the engagement structure can have a triangle shape that would fit into the inverted triangle shape. In another example, if the slot has a shape of a half circle, the engagement structure can have a shape of a half circle.

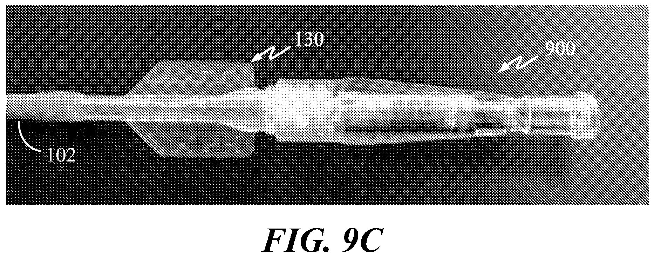

FIG. 9A is a side view of a transfer/removal tool (TR tool) 900 in accordance with some embodiments of the present disclosure. Tool 900 can be used with all embodiments of implants and implant delivery systems described herein. FIG. 9B is an exploded view of the TR tool 900, and FIG. 9C is a side view of TR tool 900 attached to a proximal hub 130 of a catheter 102. FIGS. 9A-9C will be discussed concurrently. TR tool 900 is an accessory that is provided to facilitate the introduction and removal of an implant through multiple kinds of catheter hubs from different manufacturers.

Tool 900 includes a housing 915 in a which an introducer shaft 902 is slidable and at least partially located. A tool base 917 can be secured to housing 915 such that a spring shaft component 912 and a spring 910 are held therein in contact with a hub 920 of shaft 902. A lumen 922 passes through tool 900 (a first port of base 917, base 917, shaft 912, shaft 902, and an opposite port at distal terminus of shaft 902) from a proximal end (at left of FIG. 9C) to a distal end (at right of FIG. 9C) and permits movement of a pusher and implant through tool 900.

This tool is particularly suited to example embodiments of implants that utilize a passive detachment method for releasing the implant from the pusher. Such can require the junction between the implant and pusher to be held in releasable engagement by the delivery catheter until exiting the distal end of the delivery catheter, e.g., as described with respect to FIGs. 4-8C. The catheter (e.g., 102) itself maintains the coupling of the junction after transfer from the introducer into the catheter. However, prior to transfer, the coupling needs to be maintained through the tapered hub of the delivery catheter.

Tool 900 allows the implant to be loaded to a delivery catheter while maintaining the coupling between the implant and pusher. The lumen 922 is thus sized small enough to allow movement of the implant and pusher therethrough without becoming decoupled. Tool 900 is designed to attach to the catheter hub via a Luer connector (or other manner) and can work with multiple catheter models and manufacturers to maintain the coupling of the junction upon insertion. For removal, tool 900 is designed to allow the implant to be retracted far enough so the user will be able to retrieve the implant once the accessory is removed without removing the delivery catheter.

Tool 900 can include a taper 904 on a distal end 906 of introducer shaft 902 that allows mating of tool 900 with a delivery catheter hub (see, e.g., FIG. 9C). Thus, to maintain good contact and decrease the chances of decoupling, a spring assembly is provided that includes spring 910 received over spring shaft (or post) 912. Spring 910 is configured to bias against hub 920 of introducer 902 and against hub 921 of spring post 912 in the assembled tool 900 when toll 900 is fully assembled. Once the tip of insertion shaft 902 makes contact with the hub of catheter 102, taper 904 can fit into the inner taper of a catheter hub while spring 910 can push insertion shaft 902 forward and maintain constant contact with catheter hub 130. In other words, spring 910 pushes forward to enable taper 904 to maintain contact with the taper inside of the catheter hub 130. This can assist in maintaining good contact and decreasing the chances of insufficient coupling between taper 904 and the catheter hub 130, which can become problematic with geometries that differ depending on the model and manufacturer of the delivery catheter. Taper 904 is sized such that the taper at its narrowest width will contact and couple with delivery catheter hubs 130 having relatively small inner diameters, and taper 904 at its widest width will contact and couple with delivery catheter hubs 130 having relatively large inner diameters. Thus, tool 900 is couplable to catheter hubs manufactured with varying geometries by a host of manufacturers, and in this sense can be described as "universal." Tool base 917 can be a female Luer that enables connection to standard syringes.

Insertion shaft 902 is slideable along the long axis of introducer 902 and within housing 915, which can have a distally located male Luer that connects to the hub of a catheter. The range of slidable motion can be controlled by the size and length of wire 910 and the length of post 912. For example, the range of slidable motion can be increased by increasing the length of post 912 and using a longer spring. In this embodiment spring 910 is a helical spring, but other spring configurations (e.g., leaf, torsion, etc.) can be used in other embodiments of tool 900. Spring 910 can be made from an elastomer or other material with elastic properties.

In addition to use with the implant and implant delivery systems described herein, tool 900 can be used as an interface to a microcatheter that is adapted to inject a liquid embolic into the body of the patient. The microcatheter can be used to inject one or more liquid embolics together or in succession, and the embolic can be relatively more viscous than saline.

Various aspects of the present subject matter are set forth below, in review of, and/or in supplementation to, the embodiments described thus far, with the emphasis here being on the interrelation and interchangeability of the following embodiments. In other words, an emphasis is on the fact that each feature of the embodiments can be combined with each and every other feature unless explicitly stated otherwise or logically implausible.

In many embodiments, an occlusive device can include an elongate polymer body having braid coupled thereto; and a shape memory wire coupled with the polymer body.

In some embodiments, the shape memory wire can be secured to the elongate polymer body.

In some embodiments, the shape memory wire can have an instilled shape, such as a coil shape.

In some embodiments, the shape memory wire can have an instilled non-straight shape.

In any of the above embodiments, the braid can include a first and second fiber, and the shape memory wire can be located between the first and second fiber.

In any of the above embodiments, the shape memory wire can be interlaced in the braid.

In any of the above embodiments, the elongate polymer body, braid, and shape memory wire can be secured together.

In any of the above embodiments, the elongate polymer body, braid, and shape memory wire can be secured together with at least one of a band, a clamp, a cap, adhesive, or a tubular element. The elongate polymer body, braid, and shape memory wire can also be secured together with a plurality of bands, clamps, or tubular elements. The shape memory wire can be in contact with and alongside the elongate polymer body. The braid can wrap around the shape memory wire and the elongate polymer body. The shape memory wire can extend at least a partial length of the elongate polymer body. The shape memory wire can also extend a majority of the length of the elongate polymer body. The shape memory wire can also extend the entire length of the elongate polymer body. The braid can cover at least a portion of a length of the elongate polymer body. The braid can also cover an entire length of the elongate polymer body.

In any of the above embodiments, the shape memory wire may be a nickel-titanium alloy. In any of the above embodiments, the shape memory wire can also be nitinol.

In any of the above embodiments, the occlusive device can have an engagement structure at a proximal end of the occlusive device. The engagement structure can include a tube, which can be coupled to the braid. The tube can be a polymer tube, and the polymer tube can be coupled to the braid with a girth hitch knot.

In some embodiments, the engagement structure can be coupled with the braid with a fiber of the braid.

In any of the above embodiments, the polymer body comprises only a single polymer strand or a plurality of polymer strands. The polymer body can be radiopaque.

In any of the above embodiments, the shape memory wire can include a first portion and a second portion that are pre-shaped to have a first and a second shape, respectively, where the first and second shapes can be different.

In any of the above embodiments, the shape memory wire can include a first portion having a first cross-section and a second portion having a second cross-section, where the first and second cross-sections have different shapes.

In any of the above embodiments, the shape memory metal alloy can include a first portion having a first cross-section and a second portion having a second cross-section, wherein the first and second cross-sections have different sizes.

In some embodiments, the braid can include a plurality of fibers along a length of the braid, wherein the plurality of fibers are configured to induce thrombosis.

In many embodiments, a delivery system can include: an occlusive device in accordance with any of the above embodiments; an elongate tubular member having a lumen configured to receive the occlusive device; and an elongate pusher member comprising a shape memory metal alloy tube.

In some embodiments, the pusher member of the delivery system may include markings along the length to create visual and/or tactile indicators. The positioning of these indicators relative to the operator-facing end of the elongate tubular member allows the operator to approximate the position of the occlusive implant within the tubular member. This method of approximating the positioning of the occlusive implant allows shorter procedure times and reduced exposure to radiation from fluoroscopic imaging systems for both the operator and the patient.

In some embodiments, the elongate pusher member is a solid wire pusher.

In many embodiments, a method of delivering an occlusive implant can include positioning an open distal end of an elongate tubular member in proximity to a target site, where an occlusive implant is located within a lumen of the elongate tubular member. The occlusive implant can include an elongate polymer body, a braid, and a shape memory wire. The method of delivering the occlusive implant can also include deploying the occlusive implant from the elongate tubular member to the target site.

In some embodiments, the occlusive implant can have one or more features of one or more of the above embodiments. The implant can include a pusher member where the elongate tubular member can receive the pusher member within the lumen.

In some embodiments, deploying the occlusive implant can include advancing the pusher member with respect to the elongate tubular member to deploy the occlusive implant from the open distal end of the elongate tubular member. Deploying the occlusive implant can also include retracting the elongate tubular member with respect to the pusher member to deploy the occlusive implant from the open distal end of the elongate tubular member.

In many embodiments, a method of delivering occlusive implants can include positioning an open distal end of an elongate tubular member in proximity to a target site, where a first occlusive implant can be located within a lumen of the elongate tubular member. The first occlusive implant can include a first elongate polymer body, a first braid, and a shape memory wire. The method of delivery occlusive implants can further include deploying the first occlusive implant from the elongate tubular member to the target site; and deploying one or more second occlusive implants to the target site using the elongate tubular member, where the one or more second occlusive implants comprise a second elongate polymer body and a second braid. The method of delivering occlusive implants can include a first occlusive implant in accordance with any of the above embodiments.

The method of delivering occlusive implants can include a pusher member in accordance with any of the above embodiments, where the elongate tubular member can receive the pusher member within the lumen.

In many embodiments, an implant transfer tool can include: a housing coupled with a tool base; an introducer shaft having a tapered distal end and an introduce shaft hub; a spring shaft having a spring shaft hub; and a spring coupled between spring shaft hub and the insertion shaft hub, where the insertion shaft is slidable with respect to the housing and is biased towards a distal position by the spring. The tool can include a lumen sized to permit an embolic to pass through the tool.

In some embodiments, the housing can include a Luer coupling, and the tool base can include a Luer coupling.

In some embodiments, the housing is adapted to couple with a catheter adapted to introduce the embolic to the tool. The embolic can be an implant and the tool can be adapted to permit the implant coupled with a pusher to pass through the tool. The tool base can be adapted to couple with a syringe.

In some embodiments, the tapered distal end of the introducer shaft can be adapted to fit within an inner lumen of a catheter hub.

In some embodiments, the tapered distal end of the introducer shaft can be adapted to fit within inner lumens of different catheter hubs having varying diameters.

In some embodiments, the implant transfer tool can be adapted to couple with a delivery system in accordance with any of the above embodiments. The embolic can also include an occlusive device in accordance with any of the above embodiments. The embolic can be a liquid embolic. The tool can be adapted to couple with a catheter adapted to inject the liquid embolic.

As used herein and in the appended claims, the singular forms "a," "an," and "the" include plural referents unless the context clearly dictates otherwise.

Reference in the description to "one embodiment" or "an embodiment" is intended to broadly convey that a particular feature, structure, or characteristic can be used in one or more embodiments, and different instances of the term "one embodiment" or "an embodiment" broadly refer to the same or different embodiments.

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the description does not explicitly state, in a particular instance, that such combinations or substitutions are possible. It is explicitly acknowledged that express recitation of every possible combination and substitution is overly burdensome, especially given that the permissibility of each and every such combination and substitution will be readily recognized by those of ordinary skill in the art.

While the embodiments are susceptible to various modifications and alternative forms, specific examples thereof have been shown in the drawings and are herein described in detail. It should be understood, however, that these embodiments are not to be limited to the particular form disclosed, but to the contrary, these embodiments are to cover all modifications, equivalents, and alternatives falling within the spirit of the disclosure. Furthermore, any features, functions, steps, or elements of the embodiments may be recited in or added to the claims, as well as negative limitations that define the inventive scope of the claims by features, functions, steps, or elements that are not within that scope.

## Claims

1. An occlusive device comprising:
an elongate polymer body (205) including a cover (200);
a shape memory wire (210) coupled with the polymer body (205), wherein the shape memory wire (210) is secured to the elongate polymer body (205); and
an engagement structure formed at a proximal end of the elongate polymer body (205), the engagement structure comprising a tube,
**characterised in that** the tube is coupled to the cover with a girth hitch knot.

2. The occlusive device of claim 1, wherein the cover (200) is formed of a fibre.

3. The occlusive device of claim 2 wherein the fibre is braided.

4. The occlusive device of claim 1, wherein the tube is formed of a polymer.

5. The occlusive device of claim 1, wherein the cover (200) comprises a first and second fibre.

6. The occlusive device of claim 5, wherein the shape memory wire (210) is interlaced in the cover.

7. The occlusive device of claim 1, wherein the shape memory wire (210) is a shape memory metallic alloy.

8. The occlusive device of claim 7, wherein the shape memory metallic alloy is nitinol.

9. The occlusive device of claim 1, wherein the polymer body (205) comprises a single polymer strand.

10. The occlusive device of claim 1, wherein the polymer body (205) is radiopaque and shape memory.

11. The occlusive device of claim 1, wherein the shape memory wire (210) comprises a multifilament braided structure formed in an instilled non-straight form.

12. The occlusive device of claim 1, wherein the shape memory wire (210) comprises a multifilament structure comprising a first filament and a second filament, the first filament and second filament each being formed in a different shape.

## Patentansprüche

1. Verschlussvorrichtung, umfassend:
einen länglichen Polymerkörper (205) mit einer Abdeckung (200);
einen Formgedächtnisdraht (210), der mit dem Polymerkörper (205) verbunden ist, wobei der Formgedächtnisdraht (210) am länglichen Polymerkörper (205) befestigt ist; und
eine Eingriffsstruktur, die an einem proximalen Ende des länglichen Polymerkörpers (205) ausgebildet ist, wobei die Eingriffsstruktur ein Rohr umfasst,
**dadurch gekennzeichnet, dass** das Rohr mit der Abdeckung über einen Gurtknoten verbunden ist.

2. Verschlussvorrichtung nach Anspruch 1, wobei die Abdeckung (200) aus einer Faser gebildet wird.

3. Verschlussvorrichtung nach Anspruch 2, wobei die Faser geflochten wird.

4. Verschlussvorrichtung nach Anspruch 1, wobei das Rohr aus einem Polymer gebildet wird.

5. Verschlussvorrichtung nach Anspruch 1, wobei die Abdeckung (200) eine erste und eine zweite Faser umfasst.

6. Verschlussvorrichtung nach Anspruch 5, wobei der Formgedächtnisdraht (210) in der Abdeckung vernetzt ist.

7. Verschlussvorrichtung nach Anspruch 1, wobei der Formgedächtnisdraht (210) eine Formgedächtnis-Metalllegierung ist.

8. Die Verschlussvorrichtung nach Anspruch 7, wobei die Formgedächtnis-Metalllegierung Nitinol ist.

9. Verschlussvorrichtung nach Anspruch 1, wobei der Polymerkörper (205) einen einzelnen Polymerstrang umfasst.

10. Verschlussvorrichtung nach Anspruch 1, wobei der Polymerkörper (205) röntgendicht ist und Formgedächtniseigenschaften hat.

11. Verschlussvorrichtung nach Anspruch 1, wobei der Formgedächtnisdraht (210) eine geflochtene Multifilament-Struktur umfasst, die in einer eingeflößten, nicht geraden Form geformt ist.

12. Verschlussvorrichtung nach Anspruch 1, wobei der Formgedächtnisdraht (210) eine Multifilament-Struktur umfasst, die ein erstes Filament und ein zweites Filament umfasst, wobei das erste Filament und das zweite Filament jeweils in einer unterschiedlichen Form geformt sind.

## Revendications

1. Dispositif occlusif comprenant :
un corps polymère (205) allongé comportant a couvercle (200) ;
un fil à mémoire de forme (210) accouplé au corps polymère (205), dans lequel le fil à mémoire de forme (210) est fixé au corps polymère (205) allongé ; et
une structure de mise en prise formée au niveau d'une extrémité proximale du corps polymère (205) allongé, la structure de mise en prise comprenant un tube,
**caractérisé en ce que** le tube est accouplé au couvercle avec un noeud de tête d'alouette d'élingue.

2. Dispositif occlusif selon la revendication 1, dans lequel le couvercle (200) est formé d'une fibre.

3. Dispositif occlusif selon la revendication 2, dans lequel la fibre est tressée.

4. Dispositif occlusif selon la revendication 1, dans lequel le tube est formé d'un polymère.

5. Dispositif occlusif selon la revendication 1, dans lequel le couvercle (200) comprend une première et deuxième fibre.

6. Dispositif occlusif selon la revendication 5, dans lequel le fil à mémoire de forme (210) est entrelacé dans le couvercle.

7. Dispositif occlusif selon la revendication 1, dans lequel le fil à mémoire de forme (210) est un alliage métallique à mémoire de forme.

8. Dispositif occlusif selon la revendication 7, dans lequel l'alliage métallique à mémoire de forme est du nitinol.

9. Dispositif occlusif selon la revendication 1, dans lequel le corps polymère (205) comprend un brin polymère unique.

10. Dispositif occlusif selon la revendication 1, dans lequel le corps polymère (205) est radio-opaque et à mémoire de forme.

11. Dispositif occlusif selon la revendication 1, dans lequel le fil à mémoire de forme (210) comprend une structure tressée multifilament formée dans une forme non droite instillée.

12. Dispositif occlusif selon la revendication 1, dans lequel le fil à mémoire de forme (210) comprend une structure multifilament comprenant un premier filament et un deuxième filament, les premier filament et deuxième filament étant chacun formés dans une forme différente.
